# EUROPEAN PATENT SPECIFICATION

(11) **EP 1 994 926 B1**
(45) Date of publication and mention of the grant of the patent: **09.12.2009**
(21) Application number: 08104077.6
(22) Date of filing: 23.05.2008
(51) Int. Cl.: A61K 9/20, A61K 31/41

(54) **Valsartan formulations**
Valsartanformulierungen
Formulations de valsartan

(30) Priority: 24.05.2007 TR 200703568
(43) Date of publication of application: 26.11.2008
(73) Proprietor: Sanovel Ilaç Sanayi Ve Ticaret Anonim Sirketi, 80670 Istanbul (TR)
(72) Inventor: Cifter, Ümit, 80670, Istanbul (TR); Türkyilmaz, Ali, 34580, Istanbul (TR); Turp, Hasan Ali, 80670, Istanbul (TR)
(74) Representative: Sevinç, Erkan

(56) References cited:
- WO-A-2006/066961
- WO-A-2007/022113
- WO-A-2007/026261
- WO-A-2007/049291
- WO-A-2007/049292
- WO-A-2007/052307

## Description

The present invention relates to a new pharmaceutical formulation in the form of a tablet consisting of valsartan as an active agent, pregelatinized starch, microcrystalline cellulose.

### Background of the invention

Valsartan, a compound having the chemical name N-(1-oxopentyl)-N-[[2'-(1h-tetrazol-5-yl)[1,1'-biphenyl]-4-yl]methyl]-L-valine, of formula I;

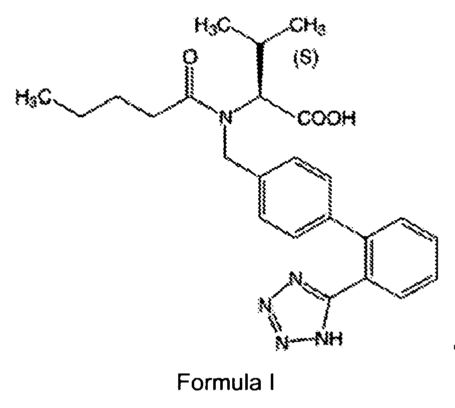

Valsartan belongs to the group of drugs that block receptors of angiotensin II and thus cause a decrease of blood pressure. Presently valsartan tablets are marketed by Novartis as DIOVAN® in doses of 40, 80, 160 and 320 mg and it is used to treat hypertension.

WO 9524901 A1 (CIBA-GEIGY AG) 07.03.1995, page 6 is directed to the use of valsartan for the treatment of diabetic nephropathy. A hard gelatine capsule comprising valsartan is disclosed.

In the international patent application WO 9524901 A1 (CIBA-GEIGY AG) 07.03.1995, example 1, a dosage capsule form is described of the following composition:

| | |
|---|---|
| Valsartan | 80.0 mg |
| Microcrystalline cellulose | 110.0 mg |
| Polyvidone K30 | 45.2 mg |
| Sodium lauryl sulphate | 1.2 mg |
| Crospovidone | 26.0 mg |
| Magnesium Stearate | 2.6 mg |

The described capsule dosage form above is prepared by below method. Valsartan and microcrystalline cellulose are granulated via wet granulation with solution of polyvidone and sodium lauryl sulphate in water. The granules are dried. Crospovidone and magnesium stearate are added to the dry granulate and the mixture is filled into capsules.

WO 9749394 A2 (NOVARTIS AG) 18.06.1997, page 14, example 1:

| | |
|---|---|
| Valsartan | 80.0 mg |
| HCTZ | 12.5 mg |
| Aerosil | 1.5 mg |
| Microcrystalline cellulose | 31.5 mg |
| Crospovidone | 20.0 mg |
| Magnesium Stearate | 4.5 mg |

The first fifth components above formulation are mixed and compacted at pressures 25 to 65 kN. The compacted material is further forced through a sieve. Granulate produced in this way is mixed with magnesium stearate and the mixture is compressed into tablets.

What is considered an extraordinary advantage of the production method of the cited application is the fact that for each specific formulation it is possible to find minimal necessary compacting pressure within the range of compacting pressures from 25 up to 65 kN, which results in obtaining a tablet having about six times faster disintegration rate than that obtained via usual compacting (i.e. using higher pressure).

WO 9749394 A2 (NOVARTIS AG) 18.06.1997, discloses compressed solid oral dosage forms, e.g., by compaction of valsartan (optionally in salt form) optionally

combined with HCTZ. In this application, the preferred range of cellulose is given 10 to 30%, e.g., 21%, for valsartan/HCTZ compositions and 5% valsartan alone. The preferred range of crospovidone is given as 10 to 20%, e.g., 13%.WO 0038676 A1 (NOVARTIS AG) 22.12.1999, page 24, lines 23-30 the application relates to a solid oral dosage form comprising valsartan as the active agent and microcrystalline cellulose wherein the weight ratio of valsartan to microcrystalline cellulose is from 2.5:1 to 0.3:1, e.g., 2:1 to 1:1, e.g., 1.4:1. This application relates to a solid oral dosage form comprising valsartan as the active agent and more than 30% of microcrystalline cellulose by weight based on the total weight of the core components of said solid oral dosage form e.g., 31 to 65%, e.g., 50%.

WO 0038676 A1 page 24, lines 15-21 it has been found surprisingly that it is possible to improve the bioavailability characteristics of known solid formulations of valsartan by increasing the proportion of microcrystalline cellulose. It has also been found surprisingly that it is possible to improve the quality, e.g., better weight uniformity and better compression for the tablets, of said known solid formulations of valsartan by decreasing proportion of crosslinked PVP.
In further aspect, this application relates to a solid oral dosage form comprising valsartan as the active agent and microcrystalline cellulose wherein the weight ratio of valsartan to microcrystalline cellulose is from 2.5:1 to 0.3:1, e.g., 2:1 to 1:1, e.g., 1.4:1

WO 2005041941 A2 (ZENTIVA A.S.) 02.11.2004, page 5, lines 26-32 and page 6, lines 1-5 the tablet material described in this application includes, apart from the valsartan active substance, optionally valsartan in combination with HCTZ, other additives, of which the most important one is suitably selected filler, which has a decisive importance for quality of the produced tablets. For ensuring the function of direct tabletting it is necessary to select filler having a defined particle size and in defined amount.

A preferable composition of the filler according to the invention WO 2005041941 A2 is microcrystalline cellulose having a particle size of 10 to 1000µm, preferably 50 to 190µm, especially preferably 90µm, in amounts above 40 to 60% by weight, spray-dried anhydrous lactose having a particle size of 10 to 250µm, preferably 150 to 250µm, in amounts of 30 to 60%, compact lactose hydrate having particle size of 10 to 250µm, in amounts 40 to 60% by weight, a polyalcohol selected from mannitol or
- microcrystalline cellulose and pregelatinized starch in a weight ratio of between 1:1 and 5:1,
- colloidal silicon dioxide,
- magnesium stearate

the tablet being preparable by directly compressing the ingredients at a pressure of 90 N to 270 N.

In a further embodiment, the tabletting mixture includes substances that improve its flow properties. Microcrystalline cellulose and pregelatinized starch are the most advantageous substance for the described mixture in this invention; preferably in a weight ratio of microcrystalline cellulose to pregelatinized starch is from 1 to 5. This ratio is calculated by dividing the weight of microcrystalline cellulose to the weight of pregelatinized starch. Ratio of these substances is important for avoiding fluctuations of the tablet weight, caused by inappropriate flow of the solid mixture through the hopper into the high performance tabletting machine.

The tablet has 80 mg to 320 mg valsartan. The tablet contains one or more filling and/or disintegrating agents. These agents are useful to produce tablets of a certain size and to support flowability step. The filling and/or disintegrating agents are microcrystalline cellulose and pregelatinized starch. The tablet also contains one or more lubricant or glidant. Lubricants and glidants are well known in the state of the art. Among them, the lubricant(s) are selected from the group of stearate, preferably magnesium stearate. The glidant(s) are selected from the group of silicon dioxide, preferably colloidal silicon dioxide.

In a further aspect, invention relates a pharmaceutical formulation in the form of a tablet consisting of;
20 to 34% of valsartan
40 to 60% of microcrystalline cellulose
8 to 40% of pregelatinized starch
0.5 to 1.5% of colloidal silicon dioxide
1.5 to 4.0% of magnesium stearate.

The tablets that described in examples are produced by direct compression techniques. Firstly, valsartan, microcrystalline cellulose, pregelatinized starch are mixed. Colloidal silicon dioxide are added to this powder and mixed. Then magnesium stearate are added to this powder and mixed. Finally, mixture is compressed by tablet compression machine.

In a further aspect, the present invention relates to tablet comprising valsartan as the active agent, microcrystalline cellulose and pregelatinized starch wherein the weight ratio of microcrystalline cellulose to pregelatinized starch is from 1 to 5.

To overcome the flowability problem and sensitivity to humidity of valsartan the tablets have high microcrystalline cellulose and pregelatinized starch rate.

### Example 1

| | |
|---|---|
| (MCC: PS=5.0: 1) | |
| Valsartan | 80.0 mg |
| Microcrystalline cellulose (MCC) | 175.0 mg |
| Pregelatinized starch (PS) | 35.0 mg |
| Colloidal silicon dioxide | 2.5 mg |
| Magnesium stearate | 7.0 mg |
| | **299.5 mg** |

### Example 2

| | |
|---|---|
| (MCC: PS=3.5:1) | |
| Valsartan | 80.0 mg |
| Microcrystalline cellulose (MCC) | 125.0 mg |
| Pregelatinized starch (PS) | 35.5 mg |
| Colloidal silicon dioxide | 2.5 mg |
| Magnesium stearate | 7.0 mg |
| | **250.0 mg** |

### Example 3

| | |
|---|---|
| (MCC: PS=1:1) | |
| Valsartan | 80.0 mg |
| Microcrystalline cellulose (MCC) | 42.0 mg |
| Pregelatinized starch (PS) | 42.0 mg |
| Colloidal silicon dioxide | 2.5 mg |
| Magnesium stearate | 7.5 mg |
| | **174.0 mg** |

### Example 4

| | |
|---|---|
| (MCC: PS=4.5:1) | |
| Valsartan | 160.0 mg |
| Microcrystalline cellulose (MCC) | 315.0 mg |
| Pregelatinized starch (PS) | 70.0 mg |
| Colloidal silicon dioxide | 5.0 mg |
| Magnesium stearate | 14.0 mg |
| | **564.0 mg** |

### Example 5

| | |
|---|---|
| (MCC: PS=3.5:1) | |
| Valsartan | 160.0 mg |
| Microcrystalline cellulose (MCC) | 250.0 mg |
| Pregelatinized starch (PS) | 71.0 mg |
| Colloidal silicon dioxide | 5.0 mg |
| Magnesium stearate | 14.0 mg |
| | **500.0 mg** |

### Example 6

| | |
|---|---|
| (MCC: PS=2.5:1) | |
| Valsartan | 160.0 mg |
| Microcrystalline cellulose (MCC) | 210.0 mg |
| Pregelatinized starch (PS) | 84.0 mg |
| Colloidal silicon dioxide | 5.0 mg |
| Magnesium stearate | 15.0 mg |
| | **474.0 mg** |

### Example 7

| | |
|---|---|
| (MCC: PS=2.5:1) | |
| Valsartan | 320.0 mg |
| Microcrystalline cellulose (MCC) | 420.0 mg |
| Pregelatinized starch (PS) | 168.0 mg |
| Colloidal silicon dioxide | 10.0 mg |
| Magnesium stearate | 30.0 mg |
| | **948.0 mg** |

Above formulations containing pregelatinized starch and microcrystalline cellulose show improved physical stability in this formulation due to its ability to reduce the water activity of the formula. In addition to these benefits, the results would show the strong flow functionality of pregelatinized starch and microcrystalline cellulose.

After exhaustive testing it has been found surprisingly that it is possible to improve the flowability characteristics of known solid formulations of valsartan by increasing the proportion of microcrystalline cellulose and pregelatinized starch. It has also been found surprisingly that it is possible to improve the resistance to humidity. (Table 1)

**Table 1. Loss on Drying Results Of Stability**

| **Example No** | **Valsartan amount (mg)** | **MCC: PS** | **Loss on Drying % (3 month stability)** | **Loss on Drying % (6 month stability)** |
|---|---|---|---|---|
| 1 | 80 | 5.0 | 0.8 | 1.2 |
| | | | 0.6 | 0.9 |
| 2 | 80 | 3.5 | 1.0 | 1.3 |
| | | | 1.2 | 1.5 |
| 3 | 80 | 1.0 | 2.1 | 2.3 |
| | | | 2.5 | 2.6 |
| 4 | 160 | 4.5 | 1.3 | 1.5 |
| | | | 1.4 | 1.7 |
| 5 | 160 | 3.5 | 1.6 | 1.9 |
| | | | 2.3 | 2.7 |
| 6 | 160 | 2.5 | 2.0 | 2.0 |
| | | | 2.8 | 3.0 |
| 7 | 320 | 2.5 | 2.9 | 3.1 |
| | | | 2.7 | 3.2 |

## Claims

1. A pharmaceutical formulation in the form of a tablet consisting of
• 20 to 34% (w/w) of valsartan as active agent,
• microcrystalline cellulose and pregelatinized starch in a weight ratio of between 1:1 and 5:1,
• colloidal silicon dioxide,
• magnesium stearate
the tablet being preparable by directly compressing the ingredients at a pressure of 90 N to 270 N.

2. The tablet according to claim 1 wherein the valsartan is present in an amount of between 80 and 320 mg of valsartan.

3. The tablet according to claim 1 consisting of;
20 to 34% of valsartan
40 to 60% of microcrystalline cellulose
8 to 40% of pregelatinized starch
0.5 to 1.5% of colloidal silicon dioxide
1.5 to 4.0% of magnesium stearate.

## Patentansprüche

1. Pharmazeutische Zubereitung in der Form einer Tablette bestehend aus
• 20 bis 34% (w/w) Valsartan als aktivem Wirkstoff,
• mikrokristalliner Cellulose und modifizierter Stärke in einem Gewichtsverhältnis zwischen 1:1 und 5:1,
• kolloidalem Siliziumdioxid,
• Magnesiumstearat,
wobei die Tablette durch Direkttablettierung der Bestandteile bei einem Druck von 90 N bis 270 N hergestellt ist.

2. Tablette nach Anspruch 1, in der das Valsartan in einer Menge zwischen 80 und 320 mg Valsartan vorhanden ist.

3. Tablette nach Anspruch 1, bestehend aus:
20 bis 34% Valsartan
40 bis 60% mikrokristalliner Cellulose
8 bis 40% modifizierter Stärke
0,5 bis 1,5% kolloidalem Siliziumdioxid
1,5 bis 4,0% Magnesiumstearat.

## Revendications

1. Formulation pharmaceutique sous forme de comprimé constitué de
■ 20 à 34 % (p/p) de valsartan en tant que principe actif,
■ de la cellulose microcristalline et de l'amidon prégélatinisé dans un rapport pondéral compris entre 1:1 et 5:1,
■ du dioxyde de silicium colloïdal,
■ du stéarate de magnésium,
le comprimé pouvant être préparé par compression directe des composants à une pression de 90 N à 270 N.

2. Comprimé selon la revendication 1 où le valsartan est présent en une quantité comprise entre 80 et 320 g de valsartan.

3. Comprimé selon la revendication 1 constitué de
20 à 34 % de valsartan
40 à 60 % de cellulose microcristalline
8 à 40 % d'amidon prégélatinisé
0,5 à 1,5 % de dioxyde de silicium colloïdal
1,5 à 4,0 % de stéarate de magnésium.
